# EUROPEAN PATENT SPECIFICATION

(11) **EP 4 408 278 B1**
(45) Date of publication and mention of the grant of the patent: **18.03.2026**
(21) Application number: 21958007.3
(22) Date of filing: 27.09.2021
(51) Int. Cl.: A61B 5/145, A61B 5/05

(54) **ANALYTE DETECTION SYSTEM**
ANALYTNACHWEISSYSTEM
SYSTÈME DE DÉTECTION D'ANALYTE

(43) Date of publication of application: 07.08.2024
(73) Proprietor: Medtrum Technologies Inc., Shanghai 201203 (CN)
(72) Inventor: YANG, Cuijun, Shanghai 201203 (CN)
(74) Representative: Vogelbruch, Keang
(86) International application number: PCT/CN2021/120856
(87) International publication number: WO 2023/044889

(56) References cited:
- CN-A- 106 137 214
- CN-A- 107 095 680
- CN-A- 112 020 327
- CN-A- 113 274 001
- CN-A- 113 274 014
- US-A1- 2016 242 685
- US-A1- 2017 172 473
- US-A1- 2019 342 637

## Description

### TECHNICAL FIELD

The invention mainly relates to the field of medical devices, in particular to an analyte detection system.

### BACKGROUND

The pancreas in a normal human body can automatically monitor the blood glucose level and automatically secrete required amount of insulin/glucagon. In the body of a type 1 diabetes patient, the pancreas does not function properly and cannot produce enough insulin for the body. Therefore, type 1 diabetes is a metabolic disease caused by abnormal pancreatic function, and diabetes is a lifelong disease. At present, there is no cure for diabetes with medical technology. The onset and development of diabetes and its complications can only be controlled by stabilizing blood glucose.

Diabetics need to have their blood glucose measured before they inject insulin into the body. At present, most of the testing methods can continuously measure blood glucose level and transmit the data to a remote device in real time for the user to view. This method is called Continuous Glucose Monitoring (CGM).

The analyte detection system needs to establish communication with the outside equipment before transmitting the analyte parameter information to the outside equipment. The existing analyte detection system transmits signal to the outside equipment at interval before establishing communication with the outside equipment. If there is an outside equipment responding to the signal, the communication with the outside equipment will be established. Otherwise, the signal will be transmitted until the communication is established or the battery power is exhausted. By using the above method, the analyte detection system is in working state after delivery. On the one hand, if the signal transmitting interval is short and thus the signal transmitting frequency is high, a large amount of battery energy will be wasted and the service life of the analyte detection system will be affected. On the other hand, if the signal transmitting interval is long, communication needs to be established during the use, and the user will have a long waiting time and poor use experience.

Therefore, there is an urgent need for an analyte detection system that can communicate with outside equipment in real time while in use and reduce battery energy consumption.

US 20190342637 describes systems and methods for activating analyte sensors electronics and mentions using a lower sampling frequency for power saving and using a higher sampling frequency for accurate event detection.

### BRIEF SUMMARY OF THE INVENTION

The objective of the present application is solved by an analyte detection system having the features of claim 1. Additional features of preferable embodiments are defined by the dependent claims.

The invention discloses an analyte detection system, which comprises an auxiliary mounting device and an analyte detection device. The analyte detection device is dormant before use and transmits signal to outside equipment at the first frequency. When it's installed on the skin surface of the host through the auxiliary installer, the wake-up module wakes up the analyte detection device according to the trigger conditions and enters the working state, in the working state, the analyte detection device transmits signal to the outside equipment at the second frequency and establishes communication with the outside equipment. There, the first frequency is less than the second frequency, which improves the user experience, reduces the battery energy consumption and ensures the service life of the analyte detection device.

The embodiment of the invention discloses an analyte detection system, which comprises an auxiliary mounting device, the auxiliary mounting device comprises a housing and an auxiliary mounting module, and the auxiliary mounting module is located in the housing. The analyte detection device comprises a shell, a sensor, a transmitter, an internal circuit, a battery and a wake-up module. The sensor comprises an external part and an internal part. The internal part, the transmitter, the internal circuit, the battery and the wake-up module are located in the shell. Before use, the housing is releasably connected to the shell, and the analyte detection device is in dormant state, which transmits signal to outside equipment at the first frequency. When the analyte detection device is installed on the skin surface of the host through the auxiliary mounting module, the wake-up module wakes up the analyte detection device according to the triggering conditions and enters the working state, and transmits signal to outside equipment at the second frequency, and then establishes communication with the outside equipment after the response of the outside equipment.

According to one aspect of the invention, the wake-up module comprises a light sensing element, after the shell is separated from the housing, the external light irradiates to the light sensing element through the shell, and the triggering condition is the light change of the light sensing element.

According to one aspect of the invention, the shell comprises a light-transmitting area through which external light is irradiated to the light sensing element.

According to one aspect of the invention, the material of the shell or light-transmitting area is one of polymethyl methacrylate, polystyrene, polycarbonate or poly 4-methyl-1-pentene.

According to one aspect of the invention, the light transmittance of shell or light-transmitting area is 40%~95%.

According to one aspect of the invention, the light-transmitting area comprises at least one light-transmitting hole in which a light-transmitting film is arranged.

According to one aspect of the invention, the housing is provided with a magnetic induction element, and the wake-up module comprises a magnetic induction element.

According to one aspect of the invention, the magnetic induction element senses the magnetic field of the magnetic component, and the triggering condition is the change of the magnetic field of the magnetic induction element.

According to one aspect of the invention, the wake-up module comprises an acceleration sensor.

According to one aspect of the invention, the acceleration sensor senses the motion state of the analyte detection device, and the triggering condition is the change of motion parameters of the acceleration sensor.

According to one aspect of the invention, the first frequency is less than the second frequency.

According to one aspect of the invention, the first frequency is 0~12 times/hour, and the second frequency is 12~3600 times/hour.

According to one aspect of the invention, in the dormant state, the analyte detection device does not transmit signal to the outside equipment, while in the working state, the analyte detection device transmits signal to the outside equipment.

Compared with the prior art, the technical scheme of the invention has the following advantages:
In the analyte detection system disclosed in the invention, the analyte detection device is in dormant state before use and does not transmit signal to the outside equipment, and then installed by the auxiliary device to the skin surface, the wake-up module wakes up the analyte detection device according to triggering conditions, and then the analyte detection device enters the working state, in the working state, the analyte detection device transmits signal to the outside equipment and establishes communication with the outside equipment, improves user experience while reducing battery energy consumption and ensuring the life of analyte detection devices.

In the analyte detection system disclosed in the invention, the analyte detection device is in dormant state before use and transmits signals to outside equipment at a low first frequency, when it is installed on the skin surface of the host through the auxiliary installer, the wake-up module wakes up the analyte detection device according to the trigger conditions and enters the working state, in the working state, the analyte detection device transmits signal to the outside equipment at the high second frequency and establishes communication with the outside equipment, which can improve user experience, reduces battery energy consumption during hibernation, and ensure the service life of analyte detection device.

Further, the wake-up module comprises light sensing element, when the shell of the analyte detection device is connected to the housing of the auxiliary mounting device, the light sensing element is located in the auxiliary installer airtight space, external light does not or weakly illuminate the light sensing element. The shell is separated from the housing when the analyte detection device is mounted to the skin surface of the host through the auxiliary mounting module, external light passes the light-transmitting area and illuminates the light sensing element. The light sensing element senses the change of light, the wake-up module wakes up the analyte detection device, enters the working state, which not only reduces the battery energy consumption before use, but also enables real-time communication with outside equipment during use, increasing the user experience.

Further, the material of the shell or the light-transmitting area is one of polymethyl methacrylate, polystyrene, polycarbonate or poly 4-methyl-1-pentene, the light transmittance of which is 40%~95%, and the external light can be irradiated to the light sensing element through the shell or the light-transmitting area.

Further, the light-transmitting area comprises at least one light-transmitting hole, and the light-transmitting film is arranged in the light-transmitting hole to prevent external water droplets, dust or other dirt from entering the device and improve the reliability of analyte detection device.

Further, the wake-up module comprises magnetic induction element, and a magnetic component is provided on the housing, when the shell of the analyte detection device is connected to the housing of the auxiliary mounting device, the magnetic induction element senses the magnetic field of the magnetic component. The shell is separated from the housing when the analyte detection device is mounted to the skin surface of the host via an auxiliary mounting module, the magnetic induction element senses the change of magnetic field intensity of the magnetic component, and then the wake-up module wakes up the analyte detection device, enters the working state, and transmits signal to the outside equipment, which not only avoids the waste of battery energy before use, but also establishes real-time communication with outside equipment during use, increasing user experience.

Further, the wake-up module comprises acceleration sensor. The shell is separated from the housing when the analyte detection device is mounted to the skin surface of the host via an auxiliary mounting module, the acceleration sensor senses the change of motion state of the analyte detection device, the wake-up module wakes up the analyte detection device, enter the working state, and then transmits signal to the outside equipment. It not only avoids the waste of battery energy before use, but also can establish real-time communication with outside equipment during use, increasing the user experience.

### BRIEF DESCRIPTION OF THE DRAWINGS

FIG. 1 is a schematic diagram of the structure of the analyte detection system according to an embodiment of the invention;
FIG. 2 is a schematic diagram of the analyte detection device according to an embodiment of the invention;
FIG. 3a is a structural schematic diagram of the wake-up module of the analyte detection device comprising a sensor according to an embodiment of the invention;
FIG. 3b is a schematic diagram of the wake-up module of the analyte detection device comprising a light sensing element according to an embodiment of the invention;
FIG. 4a is a structural schematic diagram of the analyte detection system comprising magnetic component and magnetic induction element according to an embodiment of the invention;
FIG. 4b is a structural schematic diagram of the wake-up module of an analyte detection device comprising a magnetic induction element according to an embodiment of the invention;
FIG. 4c is a schematic diagram of the wake-up module of the analyte detection device comprising a magnetic induction element according to an embodiment of the invention;
FIG. 5a is a structural schematic diagram of the analyte detection system comprising an acceleration sensor according to an embodiment of the invention;
FIG. 5b is a structural schematic diagram of the wake-up module of the analyte detection device comprising an acceleration sensor according to an embodiment of the invention;
FIG. 5c is a functional schematic diagram of the wake-up module of the analyte detection device comprising the acceleration sensor to an embodiment of the invention.

### DETAILED DESCRIPTION

As mentioned above, the existing technology of analyte detection system transmits signal at intervals after delivery and if an outside equipment responds to the signal, communication is established between the system and outside equipment, otherwise, the signal will be transmitted until communication is established, or the battery runs out of energy. On the one hand, if the signal transmitting interval is short and the signal transmitting frequency is high, a large amount of battery energy will be wasted and the service life of analyte detection system will be affected. On the other hand, if the signal transmission interval is long, the user needs to wait for a long time to establish communication, and the user experience is poor.

In order to solve the problem, the present invention provides an analyte detection system, a wake-up module is provided in the analyte detection device of the system. Before use, the analyte detection device is in dormant state and transmits signal at the first frequency to the outside equipment. When the analyte detection device is installed on the skin surface of the host through the auxiliary installation module, the wake-up module wakes the analyte detection device, enters the working state, and transmits signal to the outside equipment at the second frequency, there, the first frequency is less than the second frequency.

Various exemplary embodiments of the invention will now be described in detail with reference to the attached drawings. It is understood that, unless otherwise specified, the relative arrangement of parts and steps, numerical expressions and values described in these embodiments shall not be construed as limitations on the scope of the present invention.

In addition, it should be understood that the dimensions of the various components shown in the attached drawings are not necessarily drawn to actual proportions for ease of description, e. g. the thickness, width, length or distance of some elements may be enlarged relative to other structures.

The following descriptions of exemplary embodiments are illustrative only and do not in any sense limit the invention, its application or use. Techniques, methods and devices known to ordinary technicians in the relevant field may not be discussed in detail here, but to the extent applicable, they shall be considered as part of this manual.

It should be noted that similar labels and letters indicate similar items in the appending drawings below, so that once an item is defined or described in one of the appending drawings, there is no need to discuss it further in the subsequent appending drawings.

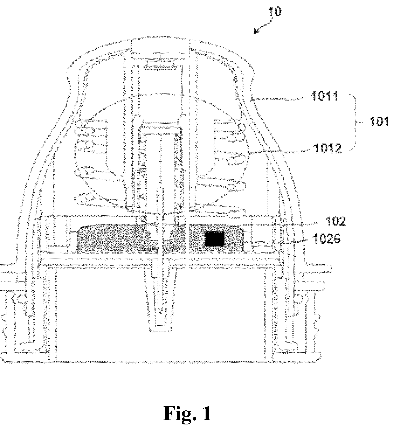

FIG. 1 is a structural diagram of the analyte detection system in an embodiment of the invention. The analyte detection system 10 comprises an auxiliary installer 101 and an analyte detection device 102. The auxiliary installer 101 comprises a housing 1011 and an auxiliary mounting module 1012, which is located inside the housing 1011. Analyte detection device 102 is located at the ejector end of auxiliary mounting module 1012, which enables rapid installation of analyte detection device 102 to the host skin surface when in use.

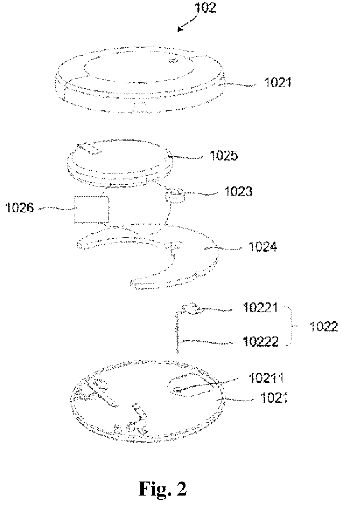

FIG. 2 is a schematic diagram of the analyte detection device to an embodiment of the invention. The analyte detection device 102 comprises a shell 1021, a sensor 1022, a transmitter 1023, an internal circuit 1024, a battery 1025 and a wake-up module 1026. Sensor 1022 comprises an external part 10221 and an internal part 10222. The external part 10221, transmitter 1023, internal circuit 1024, battery 1025 and wake-up module 1026 are located inside the shell 1021. The internal part 10222 passes through the through hole 10211 on the shell 1021 to the outside to puncture the host subcutaneous and detect the parameter information of analyte. What technicians in this field can know is that in order to pierce the internal part 10222 subcutaneous to the host, the through hole 10211 is located on the side of shell 1021 which is away from housing 1011, and at the same time, a tape (not shown in the figure) is arranged on the surface, which is used to attach the analyte detection device 102 to the skin surface of the host. The external part 10221 is electrically connected with the transmitter 1023 through the internal circuit 1024, which can transmit analyte parameter information to the outside equipment.

Before use, the shell 1021 of the analyte detection device 102 is releasable connected with the housing 1011 of auxiliary mounting device 101. Here, "releasable connection" means that shell 1021 is connected with housing 1011 by means of buckle, clamp, etc. Under the action of ejector mechanism of auxiliary mounting module 1012, the shell 1021 can be separated from housing 1011.

After the life of the sensor 1022 has expired, or the battery 1025 has run out of power, or other factors have caused the analyte detection device to fail, the user removes the entire analyte detection device from the skin surface of the host, discards it and replaces it with a new analyte detection device, is beneficial to maintain the best use of the parts of the device.

When analyte detection device 102 is installed on the skin surface of the host and starts to use, communication needs to be established with outside equipment such as PDM (Personal Diabetes Manager), mobile phone, etc., for data interaction, so as to transmit the detected analyte information data in the host to outside equipment.

As mentioned above, the analyte detection device 102 is in dormant state and transmits signal to the outside equipment at the first frequency until communication is formally established with the outside equipment. In the embodiment of the invention, the analyte detection device 102 transmits signal at a lower first frequency to an outside equipment in dormant state to reduce battery energy consumption. In the more preferred embodiment of the invention, the first frequency is 0~12 times/hour. In the more preferred embodiment of the invention, the first frequency is 0 times/hour, that is, the analyte detection device 102 does not transmit signal to the outside equipment in dormant state.

In order to establish communication between the analyte detection device 102 which is in dormant state and outside equipment, wake-up module 1026 wakes up analyte detection device 102 according to triggering conditions, so that it enters the working state and transmits signal to the outside equipment with the second frequency, and then communication is established after the outside equipment responds. The second frequency is higher than the first frequency in order to obtain analyte parameter information conveniently and in real time. In the preferred embodiment of the invention, the second frequency is 12~3600 times/hour. In a more preferred embodiment of the invention, the second frequency is 30 times/hour.

### First embodiment

### Light sensing element

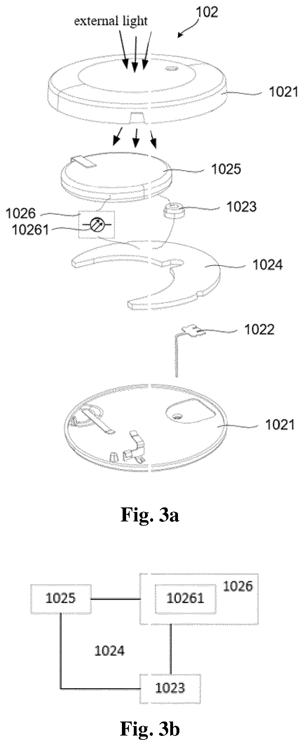

FIG. 3a is a schematic diagram of the structure of the wake-up module of the analyte detection device comprising a light sensing element in an embodiment of the invention. FIG. 3b is a functional schematic diagram of the wake-up module of the analyte detection device comprising the light sensing element in an embodiment of the invention.

In the embodiment of the invention, the wake-up module 1026 comprises a light sensing element 10261, such as photoelectric switch, which is in open state when there is no light beam or weak light beam irradiation and in a closed state when there is light beam irradiation.

In combination with Fig. 1 and Fig. 3b, transmitter 1023 is connected with battery 1025 through internal circuit 1024, forming a closed loop. The circuit is connected with a wake-up module 1026, which is connected with a light sensing element 10261 inside. The triggering condition of the wake-up module 1026 is the light intensity change received by the light sensing element 10261. In the preferred embodiment of the invention, the triggering condition of the wake-up module 1026 is that the light intensity received by the light sensing element 10261 changes from weak to strong.

In the embodiment of the invention, the analyte detection device 102 is not separated from the auxiliary mounting device 101 before it is installed on the skin surface of the host, and the shell 1021 and housing 1011 form a closed and opaque space. Since the light-transmitting area 10211 is located near the end of the housing 1011, there is no external light irradiates on light sensing element 10261, battery 1025 supplies power to transmitter 1023 through wake-up module 1026 (comprising light sensing element 10261), light sensing element 10261 is in open state, and thus the transmitter 1023 is in dormant state, and analyte detection device 102 transmits signal to outside equipment at the first frequency. After the analyte detection device 102 is installed on the skin surface of the host through the auxiliary mountine module 1012, the shell 1021 is separated from the housing 1011, and the external light can be irradiated to the light sensing element 10261 through the shell 1021. The light sensing element 10261 is in closed state. The transmitter 1023 enters the working state, and the analyte detection device 102 transmits signal to the outside equipment at the second frequency. After the response of the outside equipment, the communication is established and the analyte detection data is transmitted to the outside equipment.

In the embodiment of the invention, the shell 1021 is made of light transmittance material, such as one of polymethyl methacrylate (PMMA), polystyrene (PS), polycarbonate (PC) or poly 4-methyl-1-pentene (TPX), and the light transmittance of these material is 40%~95%. After the separation of shell 1021 and housing 1011, the external light can be irradiated on the light sensing element 10261 through the shell 1021.

In other embodiment of the invention, the shell 1021 comprises light-transmitting area 10211, the light transmittance of the light-transmitting area 10211 is higher than that of the shell 1021, so that more external light is irradiated on the light sensing element 10261, the light intensity variation of the light sensing element 10261 is increased, and the reliability of the light sensing element 10261 is improved.

In another embodiment of the invention, the light-transmitting area 10211 comprises at least one light-transmitting hole, or an array combination of several light-transmitting holes. The light-transmitting hole can make more external light illuminate on the light sensing element 10261, further increase the light intensity variation of the light sensing element 10261, and improve the reliability of the light sensing element 10261. A light-transmittance film is arranged in the light-transmitting hole (not shown in the figure out), which can prevent external water droplets, dust and other dirt from entering the analyte detection device through the light-transmitting hole and improve the reliability of the device.

In the embodiment of the invention, the light sensing element 10261 can sense visible light or invisible light, such as infrared or ultraviolet light. In the preferred embodiment of the invention, the light sensing element 10261 senses visible light so that the user can wake up the analyte detection device indoors or outdoors.

In other embodiment of the invention, the switch condition of open circuit and closed circuit of the light sensing element is low light irradiation to strong light irradiation, that is, before the separation of shell 1021 and housing 1011, weak external light is allowed to illuminate the interior of housing 1011, and the light sensing element 10261 receives weak light, but it is still in open circuit and the transmitter 1023 is in dormant state, which takes into account that the actual connection between shell 1021 and housing 1011 is not completely sealed. When the shell 1021 is separated from the housing 1011, the external light completely irradiates on the light sensing element 10261 through the shell 1021, and the light intensity received by the light sensing element 10261 becomes stronger. After reaching the set light intensity threshold, the light sensing element 10261 switches to the closed state, and the transmitter 1023 enters the working state to transmit signal to the outside equipment at the second frequency. After the response from the outside equipment, the communication is established and the analyte detection data is transmitted to the outside equipment.

### Second embodiment

### Magnetic component and magnetic induction element

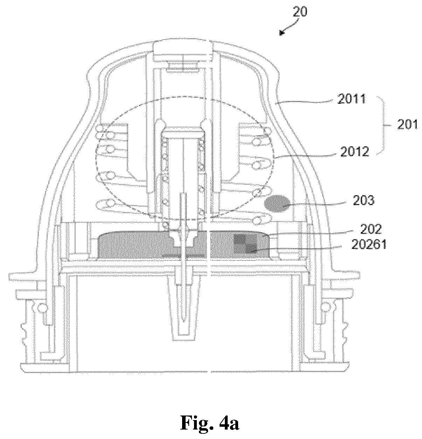

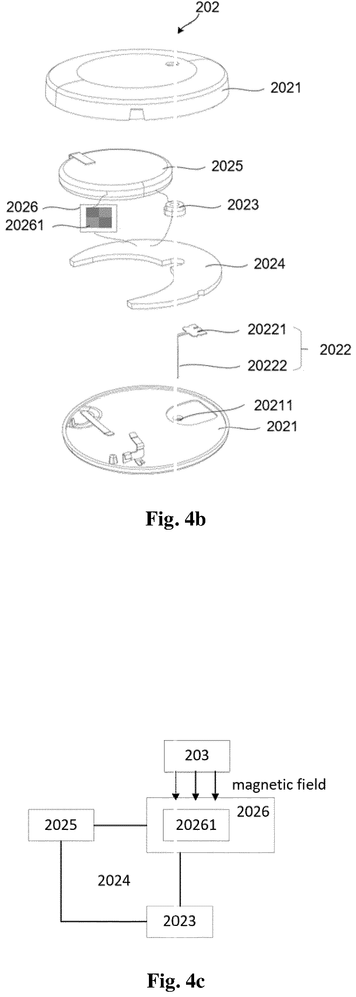

FIG. 4a is a schematic diagram of the structure of the analyte detection system comprising magnetic component and magnetic induction element in an embodiment of the invention. FIG. 4b is a schematic diagram of the structure of the wake-up module of the analyte detection device comprising the magnetic induction element in an embodiment of the invention. FIG. 4c is a schematic diagram of the function of the wake-up module of the analyte detection device comprising the magnetic induction element in an embodiment of the invention.

In the embodiment of the invention, a magnetic component 203 is arranged on the housing 2011, and a magnetic induction element 20261 is arranged in the wake-up module 2026, the battery 2025 supplies power to transmitter 2023 through the wake-up module 2026 (comprising the magnetic induction element 20261). Magnetic component 203 provides a stable magnetic field, and magnetic induction element 20261 is located in the magnetic field of magnetic component 203 and induces the magnetic field of magnetic component 203 to generate a signal. The triggering condition of the wake-up module 2026 is the magnetic field change induced by the magnetic induction element 20261.

The transmitter 2023 is connected with the battery 2025 through the internal circuit 2024, forming a closed loop, and the circuit is connected with the wake-up module 2026. Before the analyte detection device 202 is installed on the skin surface of the host, the analyte detection device 202 is not separated from the auxiliary mounting device 201, and the relative position is fixed. The magnetic field induced by the magnetic induction element 20261 to the magnetic component 203 is stable. Under the stable magnetic field, the magnetic induction element 20261 is in the open state, the transmitter 2023 is in dormant state, and analyte detection device 202 transmits signal to outside equipment at the first frequency. After the analyte detection device 202 is installed on the skin surface of the host through the auxiliary mounting module 2012, the shell 2021 is separated from the housing 2011, and the distance between the magnetic induction element 20261 and the magnetic component 203 changes, so the induced magnetic field also changes, and the magnetic induction element 20261 switches to the closed state, and transmitter 2023 enters the working state. Analyte detection device 202 transmits signal to the outside equipment at the second frequency, and then establishes communication with outside equipment after the response of the outside equipment, and transmits analyte detection data to the outside equipment.

In the embodiment of the invention, the magnetic induction element 20261 senses the magnetic field strength or magnetic field direction of the magnetic component 203. Preferably, the induction element 20261 comprises a hall element (not shown in the figure out) that sensitively sensitizes the magnetic field strength of the magnetic component 203.

In the embodiment of the invention, the magnetic component 203 may be an individual part independent of the housing 2011, or a part of the housing 2011 which is embedded in the housing 2011.

In other embodiments of the invention, the housing 2011 is embedded or enclosed with a magnetic field shielding device (not shown in the figure out), such as a Faraday cage. Technicians in this field can know that the magnetic shielding device is located outside the magnetic component 203 to reduce the impact of external magnetic field on the magnetic induction element 20261.

### Third embodiment

### Acceleration sensor

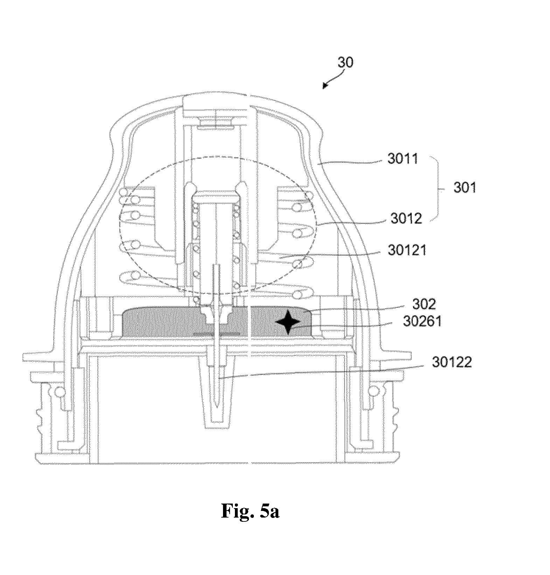

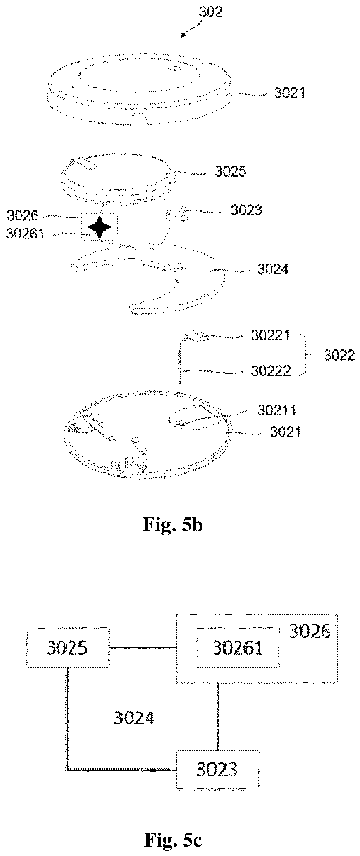

FIG. 5a is a schematic diagram of the structure of the wake-up module of the analyte detection system comprising the acceleration sensor in an embodiment of the invention. FIG. 5b is a schematic diagram of the structure of the wake-up module of the analyte detection device comprising the acceleration sensor in an embodiment of the invention. FIG. 5c is a schematic diagram of the function of the wake-up module of the analyte detection device comprising the acceleration sensor in an embodiment of the invention.

In the embodiment of the invention, the wake-up module 3026 comprises an acceleration sensor 30261, which can sensitively sense the values of motion parameters such as acceleration and adjust the circuit state of the wake-up module 3026 accordingly. The triggering condition of wake-up module 3026 is the motion parameter change of acceleration sensor 30261.

Transmitter 3023 is connected with battery 3025 through internal circuit 3024 to form a closed loop, and the circuit is connected with the wake-up module 3026, the battery 3025 supplies power to transmitter 3023 through wake-up module 3026 (comprising acceleration sensor 30261). Before the analyte detection device 302 is installed on the skin surface of the host, the analyte detection device 302 and the auxiliary mounting device 301 are relatively fixed. In order to pierce the internal part of the sensor 30222 of the analyte detection device into the skin of the host and reduce the pain sensation during the stabbing, the auxiliary mounting module 3012 adopts ejector mechanism 30121. Such as spring and other elastic parts, through the auxiliary needle 30122 can quickly pierce the body part 30222 into the host subcutaneous. When the ejector mechanism 30121 is in use, it produces a large instantaneous forward acceleration a1, and when it is installed on the skin surface of the host, it is obstructed by the skin to produce a reverse acceleration a2. After the acceleration sensor 30261 senses the above two accelerations, it can be determined that the analyte detection device 302 is installed on the skin surface of the host.

In the embodiment of the invention, before the analyte detection device 302 is installed on the skin surface of the host, the wake-up module 3026 is in an open state, and the transmitter 3023 is in a dormant state and transmits signal to the outside equipment at the first frequency. Acceleration sensor 30261 determines that the analyte detection device 302 is installed on the skin surface of the host, and the wake-up module 3026 switches to the closed state, and transmitter 3023 enters the working state and transmits signal to the outside equipment at the second frequency. After the response of the outside equipment, the communication is established and the analyte detection data is transmitted to the outside equipment.

From what has been discussed above, the embodiment of the invention discloses an analyte detection system, the analyte detection device is in dormant state before use and transmits signal to the outside equipment at the first frequency, when the analyte detection device is installed on the skin surface of the host through the auxiliary installer, the wake-up module wakes up the analyte detection device according to the triggering conditions and enters the working state, in the working state, the analyte detection device transmits signal to the outside equipment at the second frequency and establishes real time communication with the outside equipment, which can improve user experience while reducing battery energy consumption and ensuring the life of analyte detection devices.

Although some specific embodiments of the invention have been detailed through examples, technicians in the field should understand that the above examples are for illustrative purposes only and are not intended to limit the scope of the invention. Persons skilled in the field should understand that the above embodiments may be modified without departing from the scope and spirit of the present invention. The scope of the invention is limited by the attached claims.

## Claims

1. An analyte detection system (10, 20, 30), comprising:
an auxiliary mounting device comprising a housing (1011, 2011, 3011) and an auxiliary mounting module (1012, 2012, 3012), and the auxiliary mounting module (1012, 2012, 3012) is located in the housing (1011, 2011, 3011);
an analyte detection device (102, 202, 302), comprising a shell (1021, 2021, 3021), a sensor (1022, 2022, 3022), a transmitter (1023, 2023, 3023), an internal circuit (1024, 2024, 3024), a battery (1025, 2025, 3025) and a wake-up module (1026, 2026, 3026), the sensor (1022, 2022, 3022) comprises an external part (10221, 20221, 30221) and an internal part (10222, 20222, 30222), the wake-up module (1026, 2026, 3026) comprises a light sensing element (10261) which is in open state when there is no or weak light beam radiation and in closed state when there is light beam radiation, the shell (1021, 2021, 3021) comprises a light-transmitting area through which external light can irradiate to the light sensing element (10261);
the external part (10221, 20221, 30221), the transmitter (1023, 2023, 3023), the internal circuit (1024, 2024, 3024), the battery (1025, 2025, 3025) and the wake-up module (1026, 2026, 3026) are located in the shell (1021, 2021, 3021);
before using, the shell (1021, 2021, 3021) is releasable connected with the housing (1011, 2011, 3011), and the analyte detection device (102, 202, 302) is in dormant state and transmits a signal to an outside equipment at a first frequency;
when the analyte detection device (102, 202, 302) is installed on a skin surface of the host through the auxiliary mounting module (1012, 2012, 3012), the shell (1021, 2021, 3021) is separated from the housing (1011, 2011, 3011), external light irradiates to the light sensing element (10261) through the shell (1021, 2011, 3011), the wake-up module (1026, 2026, 3026) wakes up the analyte detection device (102, 202, 302) according to triggering conditions, wherein one triggering condition of the triggering conditions is the change of the external light irradiating to the light sensing element (10261), enters a working state, and transmits a signal to the outside equipment at a second frequency higher than the first frequency, after the response of the outside equipment, a communication is established between the analyte detection device (102, 202, 302) and the outside equipment.

2. Analyte detection system of claim 1, wherein a material of the shell (1021, 2021, 3021) or the light-transmitting area is one of polymethyl methacrylate, polystyrene, polycarbonate and poly 4-methyl-1-pentene.

3. Analyte detection system of claim 2, wherein a light transmittance of the shell (1021, 2021, 3021) or the light-transmitting area is 40%~95%.

4. Analyte detection system of claim 1, wherein the light-transmitting area comprises at least one light-transmitting hole in which a light-transmittance film is arranged.

5. Analyte detection system of claim 1, wherein the housing (1011, 2011, 3011) is provided with a magnetic component (203), and the wake-up module (1026, 2026, 3026) comprises a magnetic induction element (20261).

6. Analyte detection system of claim 5, wherein the magnetic induction element (20261) senses the magnetic field of the magnetic component (203), and one of the triggering conditions is the change of the magnetic field sensed by the magnetic induction element (20261).

7. Analyte detection system of claim 1, wherein the wake-up module (1026, 2026, 3026) comprises an acceleration sensor (30261).

8. Analyte detection system of claim 7, wherein the acceleration sensor (30261) senses the motion state of the analyte detection device (102, 202, 302), and one the triggering conditions is the change of a motion parameter of the analyte detection device (102, 202, 302).

9. Analyte detection system of anyone of claim 1 to 8, wherein the first frequency is 0~12 times/hour, the second frequency is 12-3600 times/hour.

## Patentansprüche

1. Analytnachweissystem (10, 20, 30), umfassend:
eine Hilfsbefestigungsvorrichtung mit einem Gehäuse (1011, 2011, 3011) und einem Hilfsbefestigungsmodul (1012, 2012, 3012), wobei das Hilfsbefestigungsmodul (1012, 2012, 3012) in dem Gehäuse (1011, 2011, 3011) angeordnet ist;
eine Analytnachweisvorrichtung (102, 202, 302), umfassend eine Hülle (1021, 2021, 3021), einen Sensor (1022, 2022, 3022), einen Sender (1023, 2023, 3023), eine interne Schaltung (1024, 2024, 3024), eine Batterie (1025, 2025, 3025) und ein Aufweckmodul (1026, 2026, 3026), wobei der Sensor (1022, 2022, 3022) einen externen Teil (10221, 20221, 30221) und einen internen Teil (10222, 20222, 30222) umfasst, wobei das Aufweckmodul (1026, 2026, 3026) ein Lichtsensorelement (10261) umfasst, das bei keiner oder schwacher Lichtstrahlung in einem offenen Zustand und bei vorhandener Lichtstrahlung in einem geschlossenen Zustand ist, wobei die Hülle (1021, 2021, 3021) einen lichtdurchlässigen Bereich aufweist, durch den externes Licht auf das Lichtsensorelement (10261) einstrahlen kann;
wobei der externe Teil (10221, 20221, 30221), der Sender (1023, 2023, 3023), die interne Schaltung (1024, 2024, 3024), die Batterie (1025, 2025, 3025) und das Aufweckmodul (1026, 2026, 3026) in der Hülle (1021, 2021, 3021) angeordnet sind;
wobei vor der Verwendung die Hülle (1021, 2021, 3021) lösbar mit dem Gehäuse (1011, 2011, 3011) verbunden ist und sich die Analytnachweisvorrichtung (102, 202, 302) in einem Ruhezustand befindet und mit einer ersten Frequenz ein Signal an ein externes Gerät überträgt;
wobei, wenn die Analytnachweisvorrichtung (102, 202, 302) mittels des Hilfsbefestigungsmoduls (1012, 2012, 3012) auf einer Hautoberfläche eines Wirts angebracht wird, die Hülle (1021, 2021, 3021) von dem Gehäuse (1011, 2011, 3011) getrennt wird, externes Licht durch die Hülle (1021, 2021, 3021) auf das Lichtsensorelement (10261) einstrahlt, das Aufweckmodul (1026, 2026, 3026) die Analytnachweisvorrichtung (102, 202, 302) gemäß Auslösebedingungen aufweckt, wobei eine der Auslösebedingungen eine Änderung des externen Lichts ist, das auf das Lichtsensorelement (10261) einstrahlt, sodass die Analytnachweisvorrichtung (102, 202, 302) in einen Arbeitszustand übergeht und mit einer zweiten Frequenz, die höher ist als die erste Frequenz, ein Signal an das externe Gerät überträgt, und nach einer Rückmeldung des externen Geräts eine Kommunikation zwischen der Analytnachweisvorrichtung (102, 202, 302) und dem externen Gerät aufgebaut wird.

2. Analytnachweissystem nach Anspruch 1, wobei ein Material der Hülle (1021, 2021, 3021) oder des lichtdurchlässigen Bereichs ausgewählt ist aus Polymethylmethacrylat, Polystyrol, Polycarbonat und Poly 4-methyl-1-penten.

3. Analytnachweissystem nach Anspruch 2, wobei eine Lichtdurchlässigkeit der Hülle (1021, 2021, 3021) oder des lichtdurchlässigen Bereichs 40 % bis 95 % beträgt.

4. Analytnachweissystem nach Anspruch 1, wobei der lichtdurchlässige Bereich mindestens eine lichtdurchlässige Öffnung umfasst, in der ein lichtdurchlässiger Film angeordnet ist.

5. Analytnachweissystem nach Anspruch 1, wobei das Gehäuse (1011, 2011, 3011) mit einer magnetischen Komponente (203) versehen ist und das Aufweckmodul (1026, 2026, 3026) ein magnetisches Induktionselement (20261) umfasst.

6. Analytnachweissystem nach Anspruch 5, wobei das magnetische Induktionselement (20261) das Magnetfeld der magnetischen Komponente (203) erfasst und eine der Auslösebedingungen eine Änderung des vom magnetischen Induktionselement (20261) erfassten Magnetfelds ist.

7. Analytnachweissystem nach Anspruch 1, wobei das Aufweckmodul (1026, 2026, 3026) einen Beschleunigungssensor (30261) umfasst.

8. Analytnachweissystem nach Anspruch 7, wobei der Beschleunigungssensor (30261) den Bewegungszustand der Analytnachweisvorrichtung (102, 202, 302) erfasst und eine der Auslösebedingungen eine Änderung eines Bewegungsparameters der Analytnachweisvorrichtung (102, 202, 302) ist.

9. Analytnachweissystem nach einem der Ansprüche 1 bis 8, wobei die erste Frequenz 0 bis 12 mal pro Stunde beträgt und die zweite Frequenz 12 bis 3600 mal pro Stunde beträgt.

## Revendications

1. Système de détection d'analytes (10, 20, 30), comprenant :
un dispositif de montage auxiliaire comprenant un boîtier (1011, 2011, 3011) et un module de montage auxiliaire (1012, 2012, 3012), le module de montage auxiliaire (1012, 2012, 3012) étant situé dans le boîtier (1011, 2011, 3011) ;
un dispositif de détection d'analytes (102, 202, 302), comprenant une coque (1021, 2021, 3021), un capteur (1022, 2022, 3022), un émetteur (1023, 2023, 3023), un circuit interne (1024, 2024, 3024), une batterie (1025, 2025, 3025) et un module de réveil (1026, 2026, 3026), dans lequel le capteur (1022, 2022, 3022) comprend une partie externe (10221, 20221, 30221) et une partie interne (10222, 20222, 30222), le module de réveil (1026, 2026, 3026) comprend un élément de détection de lumière (10261) qui est dans un état ouvert lorsqu'il n'y a pas ou peu de rayonnement lumineux et dans un état fermé lorsqu'il y a un rayonnement lumineux, la coque (1021, 2021, 3021) comprend une zone pénétrable à la lumière à travers laquelle la lumière externe peut irradier l'élément de détection de lumière (10261) ;
la partie externe (10221, 20221, 30221), l'émetteur (1023, 2023, 3023), le circuit interne (1024, 2024, 3024), la batterie (1025, 2025, 3025) et le module de réveil (1026, 2026, 3026) sont situés dans la coque (1021, 2021, 3021) ;
avant utilisation, la coque (1021, 2021, 3021) est reliée de manière détachable au boîtier (1011, 2011, 3011), et le dispositif de détection d'analytes (102, 202, 302) est dans un état de sommeil et émet un signal vers un équipement externe à une première fréquence ;
lorsque le dispositif de détection d'analytes (102, 202, 302) est installé sur une surface cutanée de l'hôte par l'intermédiaire du module de montage auxiliaire (1012, 2012, 3012), la coque (1021, 2021, 3021) est séparée du boîtier (1011, 2011, 3011), une lumière externe irradie l'élément de détection de lumière (10261) à travers la coque (1021, 2011, 3011), le module de réveil (1026, 2026, 3026) réveille le dispositif de détection d'analytes (102, 202, 302) selon des conditions de déclenchement, l'une des conditions de déclenchement étant le changement de la lumière externe irradiant l'élément de détection de lumière (10261), entre dans un état de travail et émet un signal vers l'équipement externe à une seconde fréquence supérieure à la première fréquence, et après la réponse de l'équipement externe, une communication est établie entre le dispositif de détection d'analytes (102, 202, 302) et l'équipement externe.

2. Système de détection d'analytes selon la revendication 1, dans lequel un matériau de la coque (1021, 2021, 3021) ou de la zone pénétrable à la lumière est l'un parmi le polyméthacrylate de méthyle, le polystyrène, le polycarbonate ou le poly-4-méthyl-1-pentène.

3. Système de détection d'analytes selon la revendication 2, dans lequel une perméabilité à la lumière de la coque (1021, 2021, 3021) ou de la zone pénétrable à la lumière est de 40 % à 95 %.

4. Système de détection d'analytes selon la revendication 1, dans lequel la zone pénétrable à la lumière comprend au moins un trou pénétrable à la lumière dans lequel est disposé un film pénétrable à la lumière.

5. Système de détection d'analytes selon la revendication 1, dans lequel le boîtier (1011, 2011, 3011) est pourvu d'un composant magnétique (203), et le module de réveil (1026, 2026, 3026) comprend un élément d'induction magnétique (20261).

6. Système de détection d'analytes selon la revendication 5, dans lequel l'élément d'induction magnétique (20261) détecte le champ magnétique du composant magnétique (203), et l'une des conditions de déclenchement est le changement du champ magnétique détecté par l'élément d'induction magnétique (20261).

7. Système de détection d'analytes selon la revendication 1, dans lequel le module de réveil (1026, 2026, 3026) comprend un capteur d'accélération (30261).

8. Système de détection d'analytes selon la revendication 7, dans lequel le capteur d'accélération (30261) détecte l'état de mouvement du dispositif de détection d'analytes (102, 202, 302), et l'une des conditions de déclenchement est le changement d'un paramètre de mouvement du dispositif de détection d'analytes (102, 202, 302).

9. Système de détection d'analytes selon l'une quelconque des revendications 1 à 8, dans lequel la première fréquence est de 0 à 12 fois/heure, et la seconde fréquence est de 12 à 3600 fois/heure.
